# FASCICULE DE BREVET EUROPEEN

(11) **EP 0 979 646 B1**
(45) Date de publication et mention de la délivrance du brevet: **30.10.2002**
(21) Numéro de dépôt: 99401998.2
(22) Date de dépôt: 06.08.1999
(51) Int. Cl.: A61K 7/48

(54) **Composition de maquillage ou de soin sans transfert contenant une silicone linéaire volatile**
Abriebfeste Schmink- oder Körperpflegezusammensetzung enthaltend ein flüchtiges lineares Silikon
Transfer resistant make-up or care composition containing a volatile linear silicone

(30) Priorité: 10.08.1998 FR 9810256; 04.09.1998 FR 9811065
(43) Date de publication de la demande: 16.02.2000
(73) Titulaire: L'OREAL, 75008 Paris (FR)
(72) Inventeur: Auguste, Frédéric, 94550 Chevilly-Larue (FR); Arnaud, Pascal, 94240 l'Hay-les-Roses (FR); Fouron, Jean-Yves, 75014 Paris (FR)
(74) Mandataire: Casalonga, Axel

(56) Documents cités:
- EP-A- 0 400 546
- EP-A- 0 440 542
- GB-A- 2 259 015
- US-A- 5 746 945
- DATABASE CHEMICAL ABSTRACTS [Online] STN Abrégé 125: 150 787, XP002104174 & JP 08 143426 A (SHISEIDO CO., LTD) 4 juin 1996 (1996-06-04)

## Description

La présente invention concerne une composition de maquillage ou de soin de la peau, de phanère (ongles, cils, sourcils) ou des lèvres sans transfert contenant l'association d'une silicone linéaire volatile, le décaméthyltétrasiloxane, et d'au moins un autre solvant plus volatil que la silicone. Elle concerne également l'utilisation de l'association d'une silicone linéaire volatile et d'au moins un autre solvant plus volatil que la première en tant que phase volatile dans des produits de maquillage ou de soin de la peau, de phanère (ongles, cils, sourcils) ou des lèvres, sans transfert, un procédé de préparation de tels produits de maquillage ou de soin sans transfert, ainsi qu'un procédé pour limiter le transfert d'une composition de maquillage et de soin de la peau, des phanères ou des lèvres.

La mise au point de produits de maquillage dits "sans transfert" fait actuellement l'objet de nombreuses recherches cosmétologiques. Ces produits, par exemple des fonds de teint ou des rouges à lèvres, des fards à paupières ou à joues, se distinguent par le fait qu'une fois appliqués sur la peau ou les lèvres, ils ne se déposent pas de façon notable sur les surfaces avec lesquelles ils viennent en contact (verre, tasse, cigarette, vêtement par exemple).

Une première approche pour empêcher le transfert des produits cosmétiques appliqués a consisté à les recouvrir d'une couche de produits réputés pour leur propriétés anti-adhésives, tels que des produits fluorés ou siliconés. Les formulations de ce type présentent cependant l'inconvénient d'une mauvaise cosméticité. Par exemple, le film de rouge à lèvres devient huileux et susceptible de migrer sur la peau contiguë aux lèvres et aux paupières.

Une autre possibilité pour obtenir des produits "sans transfert" consiste à utiliser des polymères ou résines siliconées en association avec des matières premières volatiles qui laissent, après évaporation de ces dernières, un film inerte résistant au transfert sur d'autres surfaces. Les matières premières volatiles utilisées sont classiquement des silicones cycliques de très faible viscosité (inférieure à 3 centistokes (3.10⁻⁶m²/s)) ou encore des isoparaffines.

De nombreuses formules cosmétiques sans transfert, notamment des rouges à lèvres et fonds de teints, utilisent actuellement comme solvant volatil principal la silicone volatile cyclique cyclotétradiméthylsiloxane, également appelée cyclométhicone D₄ ou plus brièvement D₄.

Une telle cyclométhicone est utilisée dans la demande EP-A-0 400 546.

Cette silicone cyclique, utilisée généralement en association avec des isoparaffines et en particulier avec l'isododécane, confère aux compositions d'excellentes propriétés cosmétiques et physico-chimiques (bon étalement, toucher non gras, compatibilité avec les autres constituants de la formule, vitesse d'évaporation appropriée etc.). Elle présente cependant l'inconvénient majeur et parfois rédhibitoire de posséder une température de cristallisation trop élevée.

En effet, son point de cristallisation se situant vers 18 °C, des phénomènes de cristallisation peuvent se produire à la surface de compositions de maquillage lorsque celles-ci sont conservées à basse température.

L'objectif de la présente invention a été par conséquent de trouver un solvant volatil ou une association de solvants volatils ayant des propriétés chimiques et physico-chimiques comparables à celles de la cyclométhicone D₄ mais présentant une température de cristallisation nettement inférieure à la température ambiante et permettant ainsi d'éviter les phénomènes de cristallisation.

La demanderesse a trouvé à présent qu'il était possible de remplacer la silicone volatile cyclométhicone D₄ dans des compositions de maquillage ou de soin sans transfert par l'association d'une silicone linéaire, à savoir le décaméthyltétrasiloxane, également appelé L₄, et d'un autre solvant cosmétique plus volatil que la silicone linéaire L₄. Les compositions cosmétiques sans transfert utilisant comme solvants volatils de la phase grasse la silicone linéaire L₄ et un autre solvant plus volatil que celle-ci conservent les propriétés cosmétiques intéressantes telles que le toucher non gras caractéristique des produits sans transfert, une vitesse d'évaporation suffisamment rapide et un étalement aisé et homogène.

La présente invention a donc pour objet une composition de maquillage ou de soin sans transfert comprenant, comme solvants volatils de la phase grasse, l'association du décaméthyltétrasiloxane linéaire (L₄) et d'au moins un deuxième solvant plus volatil que le décaméthyltétrasiloxane linéaire L₄.

L'invention a également pour objet l'utilisation de l'association du L₄ et d'au moins un deuxième solvant plus volatil que ce premier pour conférer des propriétés de non-transfert à des compositions de maquillage ou de soin.

Un autre objet de l'invention est un procédé de préparation de compositions de maquillage sans transfert.

Enfin, l'invention concerne encore un procédé pour limiter le transfert d'une composition de soin ou de maquillage de la peau, des phanères ou des lèvres avec une surface avec laquelle la peau, les phanères ou les lèvres sont mises en contact, consistant à introduire dans la composition l'association d'une silicone linéaire volatile et d'un autre solvant volatil ayant une vitesse d'évaporation supérieure à celle de la silicone et telle que définie ci-dessous.

La recherche d'un composé ou d'un mélange de composés en remplacement de la cyclométhicone D₄ ayant une volatilité comparable à cette dernière a nécessité la mise au point d'une méthode d'évaluation fiable des vitesses d'évaporation des solvants cosmétiques.

La détermination des vitesses d'évaporation a été réalisée de la manière suivante :

On introduit dans un cristallisoir (diamètre : 7 cm) se trouvant dans une enceinte régulée en température (25 °C) et en hygrométrie (humidité relative 50 %) 15 g du solvant ou du mélange de solvants à tester. On laisse le liquide s'évaporer librement, sans l'agiter, en assurant une ventilation par un ventilateur (SFMI tournant à 2700 tours/minute) disposé à 20 cm au dessus du cristallisoir contenant le solvant. On mesure à intervalles réguliers la masse du solvant restant dans le cristallisoir. Les vitesses d'évaporation sont exprimées en mg de solvant évaporé par unité de surface (cm²) et par unité de temps (minute).

La silicone volatile linéaire L₄ utilisée dans la présente invention comme solvant volatil en remplacement de la cyclométhicone D₄ est un tétramère de motifs diméthylsiloxy terminé par des groupes méthyle. Elle a un point de cristallisation de - 68 °C, une viscosité inférieure à 3 centistokes (3.10⁻⁶ m²/s) et une vitesse d'évaporation, mesurée dans les conditions décrites ci-dessus, égale à 0,309 mg/cm²/minute.

Une telle silicone est commercialisée par exemple par la société DOW CORNING sous la dénomination DC 200 Fluid 1,5 cSt.

Cette silicone ayant une vitesse d'évaporation nettement inférieure à celle de la cyclométhicone D₄ qui est 0,626 mg/cm²/minute, doit être utilisée en combinaison avec au moins un deuxième solvant ayant une vitesse d'évaporation au moins égale à 0,70 mg/cm²/minute et ne dépassant de préférence pas 7,0 mg/cm²/minute.

On peut citer à titre d'exemple de tels deuxièmes solvants volatils physiologiquement acceptables en particulier les alcanes linéaires ou ramifiés, les cycloalcanes, les hydrocarbures fluorés, les silicones ou les silicones modifiées et leurs mélanges. Parmi les solvants volatils préférés on peut citer le 2,2,4,4,6-pentaméthylheptane (isododécane) et le mélange d'isoparaffines vendu sous la dénomination ISOPAR E ® par la société EXXON CHEMICAL.

Dans un mode de réalisation préféré, on utilise comme deuxième solvant volatil l'isododécane de formule :

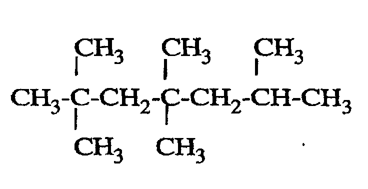

Ce composé est commercialisé par exemple sous la dénomination PERMETHYL® 99A par la société PRESPERSE Inc.

Dans les conditions de mesure spécifiées ci-dessus, sa vitesse d'évaporation est égale à 0,803 mg/cm²/minute.

Comme autre deuxième solvant volatil, on peut citer l'octaméthyltrisiloxane linéaire (L₃) qui a une vitesse d'évaporation de 2,34 mg/cm²/minute et l'hexaméthyldisiloxane (L₂). Lorsque le deuxième solvant volatil est l'octaméthyltrisiloxane, celui-ci est présent en un rapport pondéral octaméthyltrisiloxane/décaméthyltétrasiloxane inférieur à 0,4.

Les proportions respectives de décaméthyltétrasiloxane et du deuxième solvant volatil dépendent principalement de la vitesse d'évaporation de ce deuxième solvant. Plus celle-ci est élevée, plus la proportion de décaméthyltétrasiloxane est importante.

Ainsi :
- lorsque la vitesse d'évaporation du deuxième solvant, mesurée dans les conditions décrites précédemment, est comprise entre 0,70 et 0,90 mg/cm²/minute, de préférence entre 0,75 et 0,85 mg/cm²/min, autrement dit lorsque le rapport de la vitesse d'évaporation dudit deuxième solvant volatil à celle du décaméthyltétrasiloxane est compris entre 2,2 et 3 de préférence entre 2,4 et 2,8, le décaméthyltétrasiloxane est utilisé à raison de 15 à 40 % en poids, de préférence à raison de 25 à 35 % en poids, rapporté au poids total du mélange de solvants volatils ; et
- lorsque la vitesse d'évaporation du deuxième solvant, mesurée dans les conditions décrites précédemment, est comprise entre 4,0 et 7,0 mg/cm²/minute, de préférence entre 5,0 et 6,0 mg/cm²/min, autrement dit lorsque le rapport de la vitesse d'évaporation dudit deuxième solvant volatil à celle du décaméthyltétrasiloxane est compris entre 12,9 et 22,7 de préférence entre 16,1 et 19,5, le décaméthyltétrasiloxane est utilisé à raison de 75 à 95 % en poids, de préférence à raison de 85 à 95 % en poids, rapporté au poids total du mélange de solvants volatils.

Dans le cas de mélanges de solvants contenant, outre L₄, deux ou plus autres solvants plus volatils, la proportion des solvants plus volatils est généralement d'autant plus faible que la vitesse d'évaporation du mélange qu'ils forment est élevée. Parmi les mélanges ternaires préférés on peut citer les mélanges L₄/isododécane/Isopar E®(ISOPAR E® est un mélange d'isoparaffines commercialisé par la société EXXON CHEMICAL), en particulier le mélange, en pourcent en poids 91 % L₄/6 % ISOPAR E®/3 % Isododécane. On peut également citer les mélanges ternaires L₄/isododécane/L₃ pour lesquels le rapport pondéral L₃/L₄ est inférieur à 0,4.

Les compositions de maquillage ou de soin de la présente invention peuvent contenir en outre une ou plusieurs cires d'origine animale, végétale ou synthétique ayant un point de fusion supérieur à 30°C et idéalement supérieur à 45°C. Ces cires jouent généralement le rôle d'agent durcissant et/ou gélifiant. Elles sont choisis entre autres parmi la lanoline éventuellement hydrogénée, hydroxylée ou acétylée, la cire d'abeille, le spermacéti, les alcools de lanoline, les acides gras de lanoline et l'alcool de lanoline acétylée, la cire de Carnauba, de Candellila, de kapok, d'Ouricury, de riz, de jojoba hydrogénée, d'Alfa ou du Japon, les cires de fibres de liège ou de canne à sucre, le beurre de cacao, les cires de paraffine, de lignite, de pétrolatum, de vaseline ou les cires microcristallines, la cérésine, l'ozokérite, la cire de montan, les cires de polyéthylène, les cires obtenues par synthèse de Fischer-Tropsch, les esters linéaires résultant de la réaction d'un acide carboxylique saturé en C₁₀₋₄₀ et d'un alcool saturé en C₁₀₋₄₀, les alcools gras en C₂₀₋₆₀, l'alcool cétylique, l'alcool stéarylique, les lanolates ou stéarates de calcium, les huiles de ricin, de palme, de coco, de tournesol ou de coprah hydrogénées.

Les compositions de la présente invention peuvent contenir également au moins une gomme de silicone, c'est-à-dire des polysiloxanes de masse moléculaire moyenne en nombre élevée, de l'ordre de 200 000 à 1 000 000, et ayant une viscosité supérieure à 500 000 mPa.s.

La quantité de gomme de silicone est généralement inférieure à 2 % en poids, et de préférence égale à 0,1 % en poids.

Les compositions cosmétiques sans transfert peuvent comprendre en outre au moins une huile non volatile d'origine végétale, animale, minérale ou synthétique. On peut citer à titre d'exemple
- les huiles de silicone de faible viscosité telle que les polysiloxanes linéaires ayant un degré de polymérisation compris entre 3 et 2000, par exemple les polydiméthylsiloxanes de viscosité suférieure à 10 mPa.s, les phényldiméthicones, les phényltriméthicones, les polyphénylméthylsiloxanes et des mélanges de ceux-ci ;
- les huiles hydrocarbonées telles que le perhydrosqualène, les triglycérides liquides d'acides gras en C₄₋₁₀, les esters de synthèse de formule R₁COOR₂ dans laquelle R₁ représente le reste d'un acide gras supérieur en C₆₋₂₉ et R₂ une chaîne hydrocarbonée en C₃₋₃₀, les alcools gras en C₁₂₋₂₆,
- les huiles fluorées.

Ces huiles non volatiles sont présentes de préférence en une quantité comprise entre 5 et 60 % en poids de la composition totale.

Les compositions cosmétiques sans transfert peuvent contenir bien entendu également les principes actifs qui leur confèrent leur propriétés cosmétiques caractéristiques et des adjuvants cosmétiques. Il s'agit par exemple de substances telles que les filtres solaires, les agents anti-radicaux libres, les agents hydratants, les vitamines, les protéines, les céramides, les agents régulateurs de pH, les anti-oxydants, les agents conservateurs, les charges, les pigments et colorants, les émollients, les agents anti-mousse, les parfums, les agents tensioactifs et les plastifiants.

Bien entendu, l'homme de métier veillera à choisir ce ou ces éventuels composés complémentaires et leur quantité, de manière telle que les propriétés avantageuses attachées intrinsèquement à la composition cosmétique de l'invention ne soient pas, ou substantiellement pas, altérées par la ou les adjonctions envisagées.

La composition de l'invention contient avantageusement une phase particulaire généralement présente à raison de 0,05 a 35 % du poids total de la composition, de préférence de 2 à 25 %, et qui peut comprendre des pigments et/ou des nacres et/ou des charges habituellement utilisés dans les compositions cosmétiques. Cette charge peut conduire à une composition colorée, blanche ou incolore.

Par pigments, il faut comprendre des particules blanches ou colorées, minérales ou organiques, insolubles dans la phase grasse liquide, destinées à colorer et/ou opacifier le produit. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0,05 à 25 % du poids de composition finale, et de préférence à raison de 2 à 15 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (DC Red N°7), aluminium.

Les nacres peuvent être présentes dans la composition à raison de 0 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré

Les charges peuvent être présentes à raison de 0 à 35 % du poids total de la composition, de préférence 2 a 15 %. On peut notamment citer le talc, le mica, la silice, le kaolin, les poudres de Nylon (Orgasol notamment) et de polyethylene, le Téflon, l'amidon, le nitrure de bore, des microsphères de copolymères telles que l'Expancel (Nobel Industrie), le polytrap (Dow Corning) et les microbilles de résine de silicone (Tospearl de Toshiba, par exemple).

Les compositions de l'invention peuvent, en outre, contenir des colorants liposolubles et/ou des colorants hydrosolubles.

Les compositions cosmétiques de l'invention peuvent se présenter sous une forme solide, pâteuse ou liquide. Il peut s'agir d'émulsions ou de compositions anhydres. On peut citer à titre d'exemples des rouges à lèvres, des fonds de teint solides ou liquides, des mascaras, des fards à joues ou à paupières et autres produits similaires, des produits anticernes, des produits de protection solaire, de coloration de la peau ou d'hygiène corporelle (déodorant notamment).

Les procédés de fabrication des produits selon l'invention ne diffèrent en rien des procédés classiquement utilisés en cosmétique et sont parfaitement connus de l'homme de l'art.

Un produit de maquillage solide coulé tel qu'un rouge à lèvres, un fond de teint solide ou une poudre compacte sont fabriqués par exemple par
- fusion et mélange des composants non volatils de la composition,
- addition, à une température plus basse, de la phase volatile,
- coulage du mélange ainsi obtenu dans un moule de forme appropriée, et - refroidissement jusqu'à température ambiante.

L'invention est illustrée plus en détail dans les exemples suivants.

### Exemple 1 (Exemple comparatif)

On prépare un rouge à lèvres à partir des ingrédients suivants :
- Cire de polyéthylène vendue sous la dénomination
   PERFORMALENE 655 ® par la société
   NEW PHASE TECHNOLOGIES 20 % en poids
- Cyclotétradiméthylsilocane (cyclométhicone D₄) 74 % en poids
- DC Red n° 7 Calcium lake (pigment) 6 % en poids

La vitesse d'évaporation du seul solvant volatil D₄ est égale à 0,626 mg/cm²/minute dans les conditions décrites dans la description.

### Exemple 2

On prépare un rouge à lèvres à partir des ingrédients suivants :
- Cire de polyéthylène vendue sous la dénomination
   PERFORMALENE 655 ® par la société
   NEW PHASE TECHNOLOGIES 20 % en poids
- décaméthyltétrasiloxane
   (DC 200 Fluid 1,5 cSt commercialisé par
   la société DOW CORNING) 22,2 % en poids
- isododécane (PERMETHYL® 99A par la
   société PRESPERSE Inc.) 51,8 % en poids
- DC Red n°7 Calcium lake (pigment) 6 % en poids

La vitesse d'évaporation du mélange L₄/isododécane (3/7) est égale à 0,619 mg/cm²/minute dans les conditions décrites dans la description, c'est-à-dire très peu différente de celle de la cyclométhicone D₄ utilisée dans l'Exemple 1.

### Exemple 3

On prépare un rouge à lèvres à partir des ingrédients suivants :
- Cire de polyéthylène vendue sous la dénomination
   PERFORMALENE 655® par la société
   NEW PHASE TECHNOLOGIES 20 % en poids
- décaméthyltétrasiloxane (DC 200 Fluid 1,5 cSt
   commercialisé par la société DOW CORNING) 66,6 % en poids
- mélange d'isoparaffines (ISOPAR E®
   commercialisé par la société EXXON CHEMICAL) 7,4 % en poids
- DC Red n°7 Calcium lake (pigment) 6 % en poids

La vitesse d'évaporation du mélange volatil L₄/isoparaffines (9/1) est égale à 0,926 mg/cm²/minute dans les conditions décrites dans la description. On retrouve l'ordre de grandeur de l'Exemple 1.

Les rouges à lèvres des exemples 2 et 3 ne cristallisent pas lorsqu'on les conserve à basse température.

## Revendications

1. Composition de maquillage ou de soin sans transfert, **caractérisée par le fait qu'**elle comprend, en tant que solvant volatil de la phase grasse, l'association du décaméthyltétrasiloxane linéaire (L₄) et d'au moins un deuxième solvant volatil ayant une vitesse d'évaporation supérieure à celle du décaméthyltétrasiloxane linéaire, au moins égale à 0,70 mg/cm²/minute et ne dépassant pas 7,0 mg/cm²/minute.

2. Composition sans transfert selon la revendication 1, **caractérisée en ce que** ledit deuxième solvant volatil ayant une vitesse d'évaporation supérieure à celle du décaméthyltétrasiloxane est choisi parmi les alcanes linéaires ou ramifiés, les cycloalcanes, les hydrocarbures fluorés, les silicones, les silicones modifiées et leurs mélanges.

3. Composition sans transfert selon la revendication 1 ou 2, **caractérisée par le fait que** le rapport de la vitesse d'évaporation dudit deuxième solvant volatil à celle du décaméthyltétrasiloxane est compris entre 2,2 et 3, de préférence entre 2,4 et 2,8, et que le décaméthyltétrasiloxane est alors utilisé à raison de 15 à 40 % en poids, de préférence de 25 à 35 % en poids, rapporté au poids total du mélange de solvants volatils.

4. Composition sans transfert selon la revendication 1 ou 2, **caractérisée par le fait que** le rapport de la vitesse d'évaporation dudit deuxième solvant volatil à celle du décaméthyltétrasiloxane est compris entre 12,9 et 22,7, de préférence entre 16,1 et 19,5, et que le décaméthyltétrasiloxane est alors utilisé à raison de 75 à 95 % en poids, de préférence de 85 à 95 % en poids, rapporté au poids total du mélange de solvants volatils.

5. Composition sans transfert selon l'une quelconque des revendication précédentes, **caractérisée en ce que** ledit deuxième solvant volatil ayant une vitesse d'évaporation supérieure à celle du décaméthyltétrasiloxane est l'isododécane (2,2,4,4,6-pentaméthylheptane), un mélange d'isoparaffines, l'octaméthyltrisiloxane (L₃), l'hexaméthyldisiloxane (L₂) ou des mélanges de ceux-ci.

6. Composition sans transfert selon la revendication 5, **caractérisée par le fait que**, lorsque ledit deuxième solvant volatil est l'octaméthyltrisiloxane (L₃), celui-ci est présent en un rapport pondéral L₃/L₄ inférieur à 0,4.

7. Composition selon l'une quelconque des revendications 1 à 5, **caractérisée par le fait que** ledit deuxième solvant volatil ayant une vitesse d'évaporation supérieure à celle du décaméthyltétrasiloxane est un mélange de deux solvants.

8. Composition selon la revendication 7, **caractérisée par le fait que** ledit deuxième solvant volatil ayant une vitesse d'évaporation supérieure à celle du décaméthyltétrasiloxane est un mélange d'isododécane et d'une silicone volatile choisie parmi l'octaméthyltrisiloxane (L₃), l'hexaméthyldisiloxane (L₂) et des mélanges de ceux-ci.

9. Composition selon la revendication 8, **caractérisée par le fait que** lorsque ledit deuxième solvant volatil ayant une vitesse d'évaporation supérieure à celle du décaméthyltétrasiloxane est un mélange d'isododécane et d'octaméthyltrisiloxane, le rapport d'octaméthyltrisiloxane/décaméthyltétrasiloxane est inférieur à 0,4.

10. Composition sans transfert selon l'une quelconque des revendications précédentes, **caractérisée en ce qu'**elle comprend en outre une ou plusieurs cires d'origine animale, végétale ou synthétique.

11. Composition sans transfert selon l'une quelconque des revendications précédentes, **caractérisée en ce qu'**elle comprend en outre une gomme de silicone, de préférence en une quantité inférieure à 2 % en poids.

12. Composition sans transfert selon l'une quelconque des revendications précédentes, **caractérisée en ce qu'**elle comprend en outre au moins une huile non volatile d'origine végétale, animale, minérale ou synthétique, de préférence en une quantité comprise entre 5 et 60 % en poids de la composition totale.

13. Composition sans transfert selon l'une quelconque des revendications précédentes, **caractérisée en ce qu'**elle comprend en outre un ou plusieurs adjuvants ou principes actifs cosmétiques choisis parmi les filtres solaires, les agents anti-radicaux libres, les agents hydratants, les vitamines, les protéines, les céramides, les agents régulateurs de pH, les anti-oxydants, les agents conservateurs, les pigments et colorants, les émollients, les agents anti-mousse, les silicones, les parfums, les agents tensioactifs, les plastifiants et les mélanges de ces composés.

14. Composition sans transfert selon l'une quelconque des revendications précédentes, **caractérisée par le fait qu'**elle se présente sous la forme d'un produit de maquillage solide, liquide ou pâteux, anhydre ou en émulsion.

15. Composition sans transfert selon la revendication 14, **caractérisée en ce qu'**elle se présente sous la forme d'un produit de maquillage solide.

16. Composition sans transfert selon la revendication 14, **caractérisée en ce que** le produit de maquillage est un rouge à lèvres, un fond de teint, un fard à joues ou à paupières ou un mascara.

17. Utilisation de l'association du décaméthyltétrasiloxane linéaire (L₄) et d'au moins un autre solvant volatil, compatible avec le décaméthyltétrasiloxane linéaire L₄, physiologiquement acceptable et ayant une vitesse d'évaporation supérieure à celle du décaméthyltétrasiloxane linéaire L₄, au moins égale à 0,70 mg/cm²/minute et ne dépassant pas 7,0 mg/cm²/minute, en tant que solvants volatils dans des compositions de maquillage ou de soin pour conférer à ces compositions des propriétés de sans transfert.

18. Procédé pour limiter le transfert d'une composition de soin ou de maquillage de la peau, des phanères ou des lèvres avec une surface avec laquelle la peau, les phanères ou les lèvres sont mises en contact, consistant à introduire dans la composition l'association telle que définie aux revendications 1 à 9.

## Patentansprüche

1. Zusammensetzung zum Schminken oder zur Pflege ohne Transfer, **dadurch gekennzeichnet, daß** sie als flüchtiges Lösungsmittel der Fettphase das lineare Decamethyltetrasiloxan L₄ in Kombination mit mindestens einem zweiten flüchtigen Lösungsmittel enthält, das eine Verdampfungsgeschwindigkeit, die über der Verdampfungsgeschwindigkeit des linearen Decamethyltetrasiloxans liegt, von mindestens 0,70 mg/cm²/min und nicht über 7,0 mg/cm²/min aufweist.

2. Zusammensetzung ohne Transfer nach Anspruch 1, **dadurch gekennzeichnet, daß** das zweite flüchtige Lösungsmittel, das eine Verdampfungsgeschwindigkeit über der Verdampfungsgeschwindigkeit von Decamethyltetrasiloxan hat, unter den geradkettigen oder verzweigten Alkanen, Cycloalkanen, fluorierten Kohlenwasserstoffen, Siliconen, modifizierten Siliconen und deren Gemischen ausgewählt ist.

3. Zusammensetzung ohne Transfer nach Anspruch 1 oder 2, **dadurch gekennzeichnet, daß** das Verhältnis der Verdampfungsgeschwindigkeit des zweiten flüchtigen Lösungsmittels und der Verdampfungsgeschwindigkeit von Decamethyltetrasiloxan im Bereich von 2,2 bis 3 und vorzugsweise 2,4 bis 2,8 liegt und dadurch, daß das Decamethyltetrasiloxan in einer Menge von 15 bis 40 Gew.-% und vorzugsweise 25 bis 35 Gew.-%, bezogen auf das Gesamtgewicht des Gemisches der flüchtigen Lösungsmittel, verwendet wird.

4. Zusammensetzung ohne Transfer nach Anspruch 1 oder 2, **dadurch gekennzeichnet, daß** das Verhältnis der Verdampfungsgeschwindigkeit des zweiten flüchtigen Lösungsmittels und der Verdampfungsgeschwindigkeit von Decamethyltetrasiloxan im Bereich von 12,9 bis 22,7 und vorzugsweise 16,1 bis 19,5 liegt und dadurch, daß das Decamethyltetrasiloxan in einer Menge von 75 bis 95 Gew.-% und vorzugsweise 85 bis 95 Gew.-%, bezogen auf das Gesamtgewicht des Gemisches von flüchtigen Lösungsmitteln, verwendet wird.

5. Zusammensetzung ohne Transfer nach einem der vorhergehenden Ansprüche, **dadurch gekennzeichnet, daß** das zweite flüchtige Lösungsmittel, das eine Verdampfungsgeschwindigkeit über der Verdampfungsgeschwindigkeit von Decamethyltetrasiloxan aufweist, das Isododecan (2,2,4,4,6-Pentamethylheptan), ein Gemisch von Isoparaffinen, das Octamethyltrisiloxan (L₃), das Hexamethyldisiloxan (L₂) oder ein Gemisch dieser Verbindungen ist.

6. Zusammensetzung ohne Transfer nach Anspruch 5, **dadurch gekennzeichnet, daß**, wenn das zweite flüchtige Lösungsmittel das Octamethyltrisiloxan (L₃) ist, das L₃ in einem Gewichtsverhältnis L₃/L₄ unter 0,4 vorliegt.

7. Zusammensetzung ohne Transfer nach einem der Ansprüche 1 bis 5, **dadurch gekennzeichnet, daß** das zweite flüchtige Lösungsmittel, das eine Verdampfungsgeschwindigkeit über der Verdampfungsgeschwindigkeit von Decamethyltetrasiloxan aufweist, ein Gemisch von zwei Lösungsmitteln ist.

8. Zusammensetzung nach Anspruch 7, **dadurch gekennzeichnet, daß** das zweite flüchtige Lösungsmittel, das eine Verdampfungsgeschwindigkeit über der Verdampfungsgeschwindigkeit von Decamethyltetrasiloxan aufweist, ein Gemisch von Isododecan und einem flüchtigen Silicon ist, das unter Octamethyltrisiloxan (L₃), Hexamethyldisiloxan (L₂) und Gemischen dieser Verbindungen ausgewählt ist.

9. Zusammensetzung nach Anspruch 8, **dadurch gekennzeichnet, daß**, wenn das zweite flüchtige Lösungsmittel, das eine Verdampfungsgeschwindigkeit über der Verdampfungsgeschwindigkeit von Decamethyltetrasiloxan aufweist, ein Gemisch von Isododecan und Octamethyltrisiloxan ist, das Verhältnis Octamethyltrisiloxan/Decamethyltetrasiloxan unter 0,4 liegt.

10. Zusammensetzung ohne Transfer nach einem der vorhergehenden Ansprüche, **dadurch gekennzeichnet, daß** sie ferner ein oder mehrere Wachse tierischer, pflanzlicher oder synthetischer Herkunft enthält.

11. Zusammensetzung ohne Transfer nach einem der vorhergehenden Ansprüche, **dadurch gekennzeichnet, daß** sie außerdem einen Silicongummi vorzugsweise in einer Menge unter 2 Gew.-% enthält.

12. Zusammensetzung ohne Transfer nach einem der vorhergehenden Ansprüche, **dadurch gekennzeichnet, daß** sie außerdem mindestens ein nicht flüchtiges Öl pflanzlicher, tierischer, mineralischer oder synthetischer Herkunft vorzugsweise in einer Menge im Bereich von 5 bis 60 % des Gewichts der gesamten Zusammensetzung enthält.

13. Zusammensetzung ohne Transfer nach einem der vorhergehenden Ansprüche, **dadurch gekennzeichnet, daß** sie ferner ein oder mehrere kosmetische Zusatzstoffe oder kosmetische Wirkstoffe enthält, die unter den Sonnenschutzfiltern, Radikalfängern für freie Radikale, Hydratisierungsmitteln, Vitaminen, Proteinen, Ceramiden, pH-Reglern, Antioxidantien, Konservierungsmitteln, Pigmenten und Farbstoffen, Emollientien, Schaumverhütungsmitteln, Siliconen, Parfums, grenzflächenaktiven Stoffen, Weichmachern und Gemischen dieser Verbindungen ausgewählt sind.

14. Zusammensetzung ohne Transfer nach einem der vorhergehenden Ansprüche, **dadurch gekennzeichnet, daß** sie in Form eines festen, flüssigen oder pastösen Produktes zum Schminken, als wasserfreies Produkt oder in Emulsion vorliegt.

15. Zusammensetzung ohne Transfer nach Anspruch 14, **dadurch gekennzeichnet, daß** sie in Form eines festen Produktes zum Schminken vorliegt.

16. Zusammensetzung ohne Transfer nach Anspruch 14, **dadurch gekennzeichnet, daß** das Produkt zum Schminken ein Lippenstift, Make-up, Wangenrouge, Lidschatten oder Mascara ist.

17. Verwendung von linearem Decamethyltetrasiloxan (L₄) in Kombination mit mindestens einem weiteren flüchtigen Lösungsmittel, das mit dem linearen Decamethyltetrasiloxan L₄ kompatibel und physiologisch akzeptabel ist und das eine Verdampfungsgeschwindigkeit über der Verdampfungsgeschwindigkeit des linearen Decamethyltetrasiloxans L₄ von mindestens 0,70 mg/cm²/min und nicht über 7,0 mg/cm²/min aufweist, als flüchtige Lösungsmittel in Zusammensetzungen zum Schminken oder zur Pflege, um diesen Zusammensetzungen "ohne Transfer"-Eigenschaften zu geben.

18. Verfahren, um das Übertragen einer Zusammensetzung zur Pflege oder zum Schminken der Haut, der Hautanhangsgebilde oder der Lippen auf eine Oberfläche, mit der die Haut, die Hautanhangsgebilde oder die Lippen in Kontakt kommen, einzuschränken, das darin besteht, in die Zusammensetzung die Kombination nach einem der Ansprüche 1 bis 9 einzuarbeiten.

## Claims

1. Transfer-resistant make-up or care composition, **characterized in that** it comprises, as volatile solvent for the fatty phase, a combination of linear decamethyltetrasiloxane (L₄) and at least one second volatile solvent whose evaporation speed is greater than that of linear decamethyltetrasiloxane at least equal to 0.70 mg/cm² minute and not exceeding 7.0 mg/cm² minute.

2. Transfer-resistant composition according to Claim 1, **characterized in that** the said second volatile solvent whose evaporation speed is greater than that of decamethyltetrasiloxane is chosen from linear or branched alkanes, cycloalkanes, fluorohydrocarbons, silicones and modified silicones, and mixtures thereof.

3. Transfer-resistant composition according to Claim 1 or 2, **characterized in that** the ratio of the evaporation speed of the said second volatile solvent to that of the decamethyltetrasiloxane is between 2.2 and 3, preferably between 2.4 and 2.8, and **in that** the decamethyltetrasiloxane is thus used in a proportion of from 15 to 40% by weight, preferably from 25 to 35% by weight, relative to the total weight of the mixture of volatile solvents.

4. Transfer-resistant composition according to Claim 1 or 2, **characterized in that** the ratio of the evaporation speed of the said second volatile solvent to that of the decamethyltetrasiloxane is between 12.9 and 22.7, preferably between 16.1 and 19.5, and **in that** the decamethyltetrasiloxane is thus used in a proportion of from 75 to 95% by weight, preferably from 85 to 95% by weight, relative to the total weight of the mixture of volatile solvents.

5. Transfer-resistant composition according to any one of the preceding claims, **characterized in that** the said second volatile solvent whose evaporation speed is greater than that of decamethyltetrasiloxane is isododecane (2,2,4,4,6-pentamethylheptane), a mixture of isoparaffins, octamethyltrisiloxane (L₃) or hexamethyldisiloxane (L₂), or mixtures thereof.

6. Transfer-resistant composition according to Claim 5, **characterized in that**, when the said second volatile solvent is octamethyltrisiloxane (L₃), it is present in an L₃/L₄ weight ratio of less than 0.4.

7. Composition according to any one of Claims 1 to 5, **characterized in that** the said second volatile solvent whose evaporation speed is greater than that of decamethyltetrasiloxane is a mixture of two solvents.

8. Composition according to Claim 7, **characterized in that** the said second volatile solvent whose evaporation speed is greater than that of decamethyltetrasiloxane is a mixture of isododecane and a volatile silicone chosen from octamethyltrisiloxane (L₃) and hexamethyldisiloxane (L₂), and mixtures thereof.

9. Composition according to Claim 8, **characterized in that** when the said second volatile solvent whose evaporation speed is greater than that of decamethyltetrasiloxane is a mixture of isododecane and octamethyltrisiloxane, the octamethyltrisiloxanedecamethyltetrasiloxane ratio is less than 0.4.

10. Transfer-resistant composition according to any one of the preceding claims, **characterized in that** it also comprises one or more waxes of animal, plant or synthetic origin.

11. Transfer-resistant composition according to any one of the preceding claims, **characterized in that** it also comprises a silicone gum, preferably in an amount of less than 2% by weight.

12. Transfer-resistant composition according to any one of the preceding claims, **characterized in that** it also comprises at least one non-volatile oil of plant, animal, mineral or synthetic origin, preferably in an amount of between 5 and 60% by weight of the total composition.

13. Transfer-resistant composition according to any one of the preceding claims, **characterized in that** it also comprises one or more cosmetic active principles or adjuvants chosen from sunscreens, free-radical scavengers, hydrating agents, vitamins, proteins, ceramides, pH regulators, antioxidants, preserving agents, pigments, dyes, emollients, antifoaming agents, silicones, fragrances, surfactants, plasticizers and mixtures of these compounds.

14. Transfer-resistant composition according to any one of the preceding claims, **characterized in that** it is in the form of a solid, liquid or pasty make-up product, which is in anhydrous or emulsion form.

15. Transfer-resistant composition according to Claim 14, **characterized in that** it is in the form of a solid make-up product.

16. Transfer-resistant composition according to Claim 14, **characterized in that** the make-up product is a lipstick, a foundation, a face powder, an eyeshadow or a mascara.

17. Use of a combination of linear decamethyltetrasiloxane (L₄) and at least one other volatile solvent, which is compatible with linear decamethyltetrasiloxane L₄, which is physiologically acceptable and whose evaporation speed is greater than that of linear decamethyltetrasiloxane L₄, at least equal to 0.70 mg/cm² minute and not exceeding 7.0 mg/cm² minute as volatile solvents in make-up or care compositions, to give these compositions transfer-resistance properties.

18. Process for limiting the transfer of a make-up or care composition from the skin, superficial body growths or the lips onto a surface with which the skin, the superficial body growths or the lips come into contact, this process consisting in introducing the combination as defined in Claims 1 to 9, into the composition.
